# EUROPEAN PATENT APPLICATION

(11) **EP 4 464 236 A1**
(43) Date of publication of application: **20.11.2024**
(21) Application number: 23173332.0
(22) Date of filing: 15.05.2023
(51) Int. Cl.: A61B 5/00, A61M 16/00, A61M 15/00, G16H 20/00

(54) **METHOD OF OPERATING AN INHALATION DEVICE, INHALATION DEVICE AND INHALATION DEVICE SYSTEM FOR PROVIDING INHALATION PROCESS FEEDBACK**

(71) Applicant: Pari Medical Holding GmbH, 82319 Starnberg (DE)
(72) Inventor: FIEBIG, David, 81479 München (DE); FUCHS, Carola, 82061 Neuried (DE); FINKE, Matthias, 82152 Planegg (DE); BUCHNER, Simon, 85241 Hebersthausen (DE); HEINE, Benjamin, 80687 München (DE); HOFFMANN, Tobias, 86938 Schondorf am Ammersee (DE); STÖCKL, Carolin, 80331 München (DE); REINHART, Markus, 86919 Utting (DE)
(74) Representative: Hoffmann Eitle

(57) **Abstract**

The present disclosure invention relates to a method of operating an inhalation device that provides retrospective inhalation process feedback, a respectively configured inhalation device and a related inhalation device system preferably all for providing inhalation process feedback.

## Description

### TECHNICAL FIELD

The present disclosure and invention relate to a method of operating an inhalation device, and an inhalation device and an inhalation device system all preferably for providing inhalation process feedback for a user, patient, caregiver, and/or physician.

Further comprised in the present disclosure and invention is an inhalation method that is optionally related to the above mentioned method of operating an inhalation device, the inhalation device and an inhalation device system. Also comprised is a related computer program. Also all for preferably providing inhalation process feedback for a user, patient, caregiver, and/or physician.

### BACKGROUND

With existing medical inhalation devices and nebulisers, a user/patient breathes as he or she thinks is good when inhaling and normally gets no feedback on how the therapy was done and the physician or caregiver does not know retrospectively, if the therapy was done correctly. The common questions then are: "Did the patient breathe optimally during inhalation?", "Did the patient breathe not too hard and not too shallow or too fast?", "Did the patient inhale long enough?", "Did the patient use up the medication?", "Did the patient breathe calmly and relaxed and evenly?", "Did the patient inhale and exhale consequently through the inhalation device?" that can currently not be answered after an inhalation. Neither the patient nor the physician or a caregiver knows whether the inhalation process was conducted correctly and whether the patient needs to be trained again regarding a good breathing procedure when inhaling.

EP 2 797 652 A1 and US 10 471 122 disclose inhalation devices configured to output signals that to some extent inform a user/patient about aspects of an ongoing inhalation process. Providing information with respect thereto retrospectively is not necessarily embraced.

### SUMMARY

### Objective

It is thus an objective of the present disclosure and invention to provide devices and procedures that allow a user/patient and a physician and a caregiver to be given information on a concluded inhalation process as a basis for user/patient-training and thus optimizing future inhalation processes. Said information could be considered to be retrospective feedback on the inhalation process and its quality.

### Advantages

Based on the present disclosure and invention, a user/patient of an inhalation device and the physician or a caregiver are put in a position to assess, whether an inhalation process was done well or not so well and can also obtain detailed information about the inhalation process retrospectively.

The user/patient is informed whether she or he inhaled well or badly. The quality of the inhalation can be documented and can be made available to the physician or caregiver. The user/patient and the physician or caregiver can learn from the information given about the concluded inhalation process and improve future inhalation processes on that basis. The user/patient or caregiver then acts more confidently during future inhalation processes and may also feel somewhat safer in the process. Also, user/patients who actually know how to do an inhalation properly sometimes wish to know if the inhalation was done correctly when they were unconcentrated or distracted during the therapy. In addition, the future therapy quality can be significantly improved. In clinical trials, it can so be ensured that the inhalation was done well and that the clinical trials are meaningful.

### Solution

The above objective is achieved and the above mentioned and more advantages realized by the devices and procedures outlined in the present disclosure, in particular the method of operating an inhalation device, the inhalation device and inhalation device system described herein below. Preferably, the present invention is further defined by the appended independent claims, whereas the dependent claims define optional features and distinct embodiments.

### DETAILED DESCRIPTION

The present disclosure and invention embrace a method of operating an inhalation device that includes: a sensing unit configured to monitor an inhalation process, to measure inhalation process parameters and to collect data of and during the inhalation process, and a control unit configured to receive and process the data collected by the sensing unit and to generate a control signal for a signalling device, wherein the method comprises:
monitoring an inhalation process comprising measuring inhalation process parameters and collecting data of and during the inhalation process, generating and sending a control signal based on the collected data to a signalling unit configured to output a signal, preferably an optical, acoustic and/or a tactile signal, wherein in response to said control signal, the signalling unit outputs a first signal after the inhalation process has been concluded if retrospectively one or more inhalation process parameters was/were within a pre-set range and outputs a second signal after the inhalation process has been concluded if retrospectively one or more inhalation process parameters was/were outside a pre-set range. Preferably, the pre-set ranges are the same for each individual inhalation process parameter.

The expression "inhalation device" as used in the present disclosure and invention relates to a medical device, i.e., a medical inhalation device, that is used for the prevention and treatment of respiratory malfunctions and diseases in humans or animals, but also for improving wellbeing and as prophylaxis. Said device, in line with the skilled person's common understanding, at least comprises a mouthpiece-part through which a user/patient is able to inhale a suitably prepared medicament according to a prescription of a physician or any other suitable agent including salt water and non-prescription medicaments and any type of aerosols. "Nebulizer" devices are also embraced. The expression "inhalation device" in fact embraces all devices suitable and configured for such functionality and purpose, preferably in the medical field.

An "inhalation process" is a process carried out using the above explained "inhalation device" and is conventionally a process in which a user/patient inhales a medicament by means and through the inhalation device for a certain amount of time and during several breath cycles. The terminology "inhalation process" is a generic term that embraces several complete breaths or breath cycles, respectively, i.e., inhalations and exhalations per time period. In the context of the present disclosure and invention, the inhalation part thereof takes centre stage since it is the part of the breath/breath cycle with which a medicament or agent is inhaled and administered and thus preferably most important to be monitored and optimized. Monitoring and optimization of exhalation is by no means included here and fully embraced by the present disclosure and invention where needed. Said inhalation process usually has a beginning at which the inhalation starts and has an end at which inhalation stops. Therebetween a certain amount of time lapses in which the user/patient performs a certain number of breath cycles, i.e., inhalation and exhalation cycles depending on the specifics of the respective therapy or treatment, preferably at least 3 to 5 breaths/breath cycles. The totality of the time passed and/or the breath cycles defines the inhalation process as such and in its totality and as indicated above include inhalation and exhalation as part of a natural breath/breath cycle. The present disclosure and invention however also comprise that the inhalation process to be monitored is only part of the totality of the time elapsed and the breath cycles taken. Preferably, although in principle possible, the concepts of the present disclosure and invention are not applied to "MDI"-processes ("metered-dosed inhalation"), which do not represent an inhalation process, but normally only a single inhalation and agent burst.

The "sensing unit" is an entity that is configured to monitor an inhalation process and collect and optionally store data with respect thereto. Said "sensing unit" embraces all suitable means for that and, for example, sensors that are suitably configured to measure pertinent inhalation process parameters. The sensing unit is preferably provided and implemented within the inhalation device itself.

The "control unit" is an entity to be understood broadly and is an "IPO" (input-process-output) means that is configured to receive data, for example, from the sensing unit, process said data, optionally store the processed data and output said data in order to instruct and control other entities either of the inhalation device itself or externally connected ones. A practical example for such control unit is a microprocessor or microcomputer with respectively configured software stored and executed thereon. The control unit is optionally provided and implemented within the inhalation device itself. Alternatively, the control unit may also be part of an external device similar to the respective option for the signalling unit. The explanations given with respect thereto below then also apply to the control unit.

Equally broadly is the definition of the "signalling unit", which is an entity that is configured to output a signal/information that is preferably recognizable by human senses, for example, audible, visible and perceptible signals. As will be detailed below, said signalling unit may be integrated into the inhalation device itself and/or is part of an external entity. A more elaborate example of the signalling unit is an electronic display device, but also embraces simpler lights, speakers and/or vibrators. The first signal may be a single signal of a single type but could also be a signal sequence combined of various different signal types. Likewise the second signal may be a single signal or can also be a signal sequence. Preferably, the first signal and the second signal are distinguishable and different from each other, such that the user/patient can readily identify the different meaning of the signals. The first signal and the second signal are representative of or part of an inhalation process feedback that is to be provided retrospectively once an inhalation process has been finished. Said inhalation process feedback may be limited to only one of the first signal and the second signal, but can also include more detailed information, for example, in graphical or written or voice form to be out by the signalling unit.

The essence of the above defined operation method is that the signalling unit after an inhalation process has been completed outputs a signal to a user/patient and/or a physician and/or a caregiver that is basically representative of a retrospective assessment of the concluded inhalation process. In other words, the user/patient is given a summary of the concluded inhalation process that includes an indication as to whether the concluded inhalation process was optimal or not. An optimal inhalation process is one in which one or more significant/characteristic inhalation process parameters was within a pre-set parameter range(s), wherein said range is representative of an optimal/best range for the specific inhalation process parameters. If this was the case for at least one or more of the inhalation process parameters, then a dedicated signal is output by the signalling unit informing a user/patient about an optimal inhalation process. In return, if one or more inhalation process parameters fell outside the pre-set parameter range(s), then a different signal is output informing the user/patient that the inhalation process was not optimal and would need improvement. A wide range of possible signals outputs by the signalling unit is conceivable and part of the present disclosure and invention. For example, such signals may range from simple and different light signals output directly on or through the inhalation device but may also embrace (elaborate) graphical information or audible or voice information output by an external signalling unit, for example, when implemented in an external electronic device like a smart phone or tablet computer. In the latter case, it is conceivable that there is detailed information related to the concluded inhalation process and its parameters are output to provide the user/patient or the physician with a detailed account of the inhalation process. Naturally, the information provided can then form the basis to improve subsequence inhalation processes, by training the user/patient and optionally in consultation with the physician and or caregiver.

Finally, it is noted that the above and herein below defined operation method and processes relate to the intrinsic functionalities of an inhalation device or components of an inhalation device system. Said operation method is thus preferably independent from an actual inhalation process to be carried out by a user/patient. Thus, the operation method represents a non-therapeutic, non-diagnostic and non-surgical method of operating an inhalation device and thus does not relate to methods for treatment of the human or animal body by surgery or therapy and diagnostic methods practised on the human or animal body.

In the above method, optionally the one or more inhalation process parameter is any one - but not limited to - of the following group: respiratory minute volume, breathing frequency, peak inspiratory flow, peak expiratory flow, tidal volume, regularity of tidal volume, regularity of breathing cycles duration, total duration of aerosol production, ratio of exhalations not done through the inhalation device, ratio of exhalation duration to inhalation duration, duration of a breathing pause after inhalation and/or after exhalation and degree of nebulization of the medicament or agent.

The above listed inhalation process parameters represent parameters that the sensing unit is configured to measure and monitor and are in line with the skilled person's common technical understanding of the respective terminologies used. The parameter "total duration of aerosol production" preferably relates to the time of actual aerosol production and when a user/patient inhales disregarding the inhalation device's idle time. Alternatively, it is the actual time of aerosol production performed by the inhalation device independently of the actual inhalation process. The parameter "ratio of exhalations not done through the inhalation device" relates to exhalations not done through and via the inhalation device, for example, if the user/patient removes the inhalation device when exhaling or exhales through the nose. The parameter "ratio of exhalation duration to inhalation duration" can also be construed as the inspiration/expiration-ratio. Finally, the parameter "duration of a breathing pause" relates to the pause/break between single consecutive breaths.

Based on one or more of said parameters, the above described inhalation process feedback is generated and given. For example, if the breathing frequency during an inhalation process is within a specific numerical range considered to be optimal for this inhalation process parameter, i.e., within the pre-set range, then the first signal is output to indicate to the user/patient that the inhalation process with respect thereto or in general was optimal. On the contrary, when the breathing frequency during the inhalation process is non-optimal, i.e., falling outside the pre-set range then a respective signal, i.e., the second signal, is output by the signalling unit indicating that the concluded inhalation process was sub-optimal and may need improvement. It is embraced by the present disclosure and invention that one or more or additional or all of the above indicated parameters can be considered in the process of evaluating the quality of an inhalation process and providing respective inhalation process feedback. Also, for each of said inhalation process parameters individual pre-set ranges may be selected.

Also embraced by the present invention and disclosure is that the falling inside or outside a pre-set range may relate to the full or almost full duration of the entire inhalation process or only parts thereof. This can in turn depend on and vary from inhalation process parameter to inhalation process parameter. For example, an inhalation may be seen to be optimal only if a specific inhalation process parameter is in the optimal range throughout all or most of the duration of the inhalation process. Alternatively, for some inhalation process parameters, an inhalation process may still be seen to be optimal, if the parameter was in an optimal range for one part, for example, half of the duration of the inhalation process.

Yet further embraced is that the pre-set range not only differs per inhalation process parameter but is also user/patient-group specific and adjustable and/or also therapy- or medicament-specific and adjustable. For example, for young users/patients an optimal inhalation process is characterised by other inhalation process parameter ranges than for older users/patients. Hence, the pre-set ranges are to be adjustable so as to provide target-group specific inhalation process feedback.

In addition, in the above method preferably the signalling unit comprises or consists of any one of the following: means to generate an optical signal; preferably a light signal, means to produce an acoustic signal; preferably a sound and/or a voice signal, means to produce a tactile signal; preferably a vibration signal and an electronic display device.

It is further part of the present disclosure and invention that in the here described methods, processes and systems, a variety of means are conceivable for the signalling unit. Embraced are basically all technical means and hardware that are capable and configured to output signals that are perceivable by humans. These means can also be freely combined in order to output any conceivable signal sequence or signal cascade that can comprise any one of optical signals, acoustic signals and haptic signals. Hence, the first signal may be a single signal of a single type but could also be a signal sequence combined of various different signal types and the second signal alike.

In one option of the present disclosure and invention, the signalling unit is implemented in the inhalation device itself.

In such a case, the signalling unit is an integral part of the inhalation device itself and thus integrated therein. This in turn implies that the inhalation device itself outputs respective signals and feedback information. For that purpose, the inhalation device itself embraces any suitable hardware means for outputting such signals and information. This has the advantage that the user/patient has a single device with which an inhalation process is performed and that at the same time provides inhalation process feedback information. For example, the inhalation device may comprise as the signalling unit one or more light sources, for example LEDs that produce (different )light signals representative of the first signal and the second signal.

More specifically and as an example, the first signal is output as a green light signal and the second signal is output as a yellow or a red light signal, optionally wherein the first light signal and the second light signal appear on the housing of the inhalation device. The green light signal is then somewhat intuitively indicating an optimal inhalation process, whereas the yellow or red light signal intuitively informs or alerts the user/patient or physician or caregiver about a non-optimal inhalation process that may require improvement. Hence, the feedback information is conveyed by means of deliberately chosen light signals and light colours. Optionally, the light signals can be continuous or discontinuous with respect to their output time, i.e. a steady or blinking light signal. This way colour-blind users/patients or caregivers can still distinguish the different meanings of the light signals.

Another option embraced by the present disclosure and invention is that the signalling unit is implemented in a device different from and external to the inhalation device, preferably in an electronic device further preferably with a computer program stored on the electronic device configured to generate a graphical user interface for outputting data of the inhalation process.

In such a case, the respective signals are output and the respective inhalation process feedback provided on a device that is not the inhalation device itself, but a device separate and external thereto. Preferably envisaged are external electronic devices like mobile phones, smart phones, tablet- and mobile computers in any kind of smart personal electronic devices. All of the devices commonly include display devices, processors and suitable computer programs that enable said devices to output information including the first and second signal, but also additional optional information on the inhalation process. For example, it is conceivable that on the display devices of the aforementioned electronic devices inhalation process feedback is provided by suitable graphical or written information. Also conceivable is a respective output in the form of sound or voice via suitable hardware means related to the electronic- or electronic display device. In principle, inhalation process feedback can be provided by all types of human-perceivable signals that the respective electronic devices can generate. As part of the present disclosure and invention it is also foreseen that the above options of having an integral and non-integral, i.e., external signalling unit are combinable or combined. For example, it is surely embraced herein that on the one hand the inhalation device itself provides a basic inhalation process feedback, whereas the signalling unit in from of or as part of an external electrical device additionally provides the inhalation process feedback in the same or in a different form and even further details. The letter is due to the fact, that the aforementioned electronic devices normally have larger and more versatile signal output capabilities than the ones that could be incorporated within the inhalation device itself. Finally, it is clear that for the above option where the signalling unit is an external unit that from a hardware perspective respective technical means are present that allow the exchange of data between the sensing unit, the control unit and the signalling unit. All technical means conceivable by the skilled person for said purpose are embraced here.

More specifically and preferably and as already indicated above, the signalling unit is an electronic display device and the operating method then further comprises: in response to said control signal and after the inhalation process, the electronic display device displays summary and feedback information regarding the concluded inhalation process, wherein the displayed information is to inform a user of the inhalation device about the quality of the concluded inhalation process, preferably in total and in respect to each of the measured parameters, and/or ways to improve the next inhalation process and/or to recommend an inhalation process guidance function for the next inhalation process.

Said further examples of the present disclosure and invention highlight again that in particular in the case where the signalling unit is an external unit and the signal output is performed on said external unit which is preferably an electronic device and more preferably an electronic display device, the degrees of freedom of the signals to the output as the inhalation process feedback are thus high. Hence, the first and/or the second signal can be elaborate signals and clearly go beyond simple light or sound signals and can basically embrace any suitable information form output within the technical capabilities of an electronic display device or more generally an electronic device including an electronic display device but also including respective other signal devices, for example standard light devices. As a preferred example, the inhalation feedback information provided can also include a reference for the user/patient to improve certain aspects of the inhalation process, specifically the ones found to be non-optimal, but also to conduct guided breathing and inhalation procedures supported by the electronic device. Further optionally, the displaying of feedback information is sorted according to the most important to the least important inhalation process parameters to be used for feedback and evaluation or sorted according to the individually worst performed inhalation process parameter to the less badly performed inhalation process parameters. For ease of use or motivation of the patient some of the not optimal parameters might be neglected to help the patient to focus on the most important inhalation process parameter to be optimized.

A further important aspect of the present disclosure and invention is an inhalation device itself comprising: a sensing unit configured to monitor an inhalation process and to measure and collect data of and during the inhalation process, a signalling unit configured to output a signal, preferably an optical, acoustic and/or a tactile signal, a control unit configured to receive and process the data collected by the sensing unit and to control the signalling unit based on said data, wherein the control unit is further configured to control the signalling unit such that the signalling unit outputs a first signal after the inhalation process has been concluded if retrospectively one or more inhalation process parameters was/were within a pre-set range and outputs a second signal after the inhalation process has been concluded if retrospectively one or more inhalation process parameters was/were outside a pre-set range.

Optionally, the above defined inhalation device is the inhalation device used in the methods and procedures according to the present disclosure and invention. In particular so for the scenario where the signalling unit is incorporated in the inhalation device itself. As it can be understood from the above defined inhalation device, preferably all of the respective components, namely the sensing unit, the control unit and the signalling unit are part of the inhalation device itself and comprised therein. Consequently, the inhalation device itself is configured and capable to generate and output the first and the second signal and to provide inhalation process feedback information. Furthermore, if the methods and procedures according to the present disclosure and invention are carried out using the above defined inhalation device, the respective signal output is performed by the inhalation device itself.

As a specific non-limiting example, the first signal is a green light signal and the second signal is a yellow or red light signal, optionally wherein the first light signal and the second light signal appear on the housing of the inhalation device. As another specific non-limiting example, the first signal is a continuous light signal and the second signal is a intermittent or blinking signal, optionally wherein the first light signal and the second light signal appear on the housing of the inhalation device. Surely, all other types of signals and signal sequences are conceivable. The signalling unit within the inhalation device may also alternatively or additionally output acoustic and/or haptic signals as the first and second signal. This option is analogous to the above described option for the operating method and the explanations provided there apply mutatis mutandis here.

Preferably, the one or more inhalation process parameter is any one of the following group: respiratory minute volume, breathing frequency, peak inspiratory flow, tidal volume, regularity of tidal volume, regularity of breathing cycles duration, total duration of aerosol production, ratio of exhalations not done through the inhalation device, ratio of exhalation duration to inhalation duration, duration of a breathing pause after the inhalation and/or the exhalation and degree of nebulization of the medicament or agent. This option is analogous to the above described option for the operating method and the explanations provided there apply mutatis mutandis here.

Further preferably, the signalling unit comprises any one of the following accommodated within the inhalation device: means to generate an optical signal; preferably a light signal, means to produce an acoustic signal; preferably a sound signal and/or a voice signal and means to produce a tactile signal; preferably a vibration signal and an electronic display device. This option is analogous to the above described option for the operating method and the explanations provided there apply mutatis mutandis here.

A yet further important aspect of the present disclosure and invention is an inhalation device system, comprising: an inhalation device, and an electronic device that is different from and separate to the inhalation device, preferably a mobile phone, smartphone, smart watch, smart personal device or tablet computer, wherein both the inhalation device and the electronic device comprise communication means configured to establish a wired or wireless communication connection between the inhalation device and the electronic device to exchange data on an inhalation process performed and concluded with the inhalation device. The electronic device can be used by the user/patient using the inhalation device or could be used remotely by another person, which could be a caregiver, physician, therapist or anybody else to get feedback about the user's/patient's inhalation without the need to be physically near to the user/patient, which is also beneficial in scenarios of infectious diseases or the like.

Optionally, the above defined inhalation device system comprises a medical inhalation device and preferably the inhalation device that has been outlined herein above. Yet further optionally, the above defined inhalation device system is a device-related representation of the above described option of having the signalling unit external to the inhalation device itself. Also, all of the above outlined and explained interrelations, functionalities and technical advantages and benefits apply mutatis mutandis to the inhalation device system and will not be repeated here for the sake of conciseness. The present inhalation device system basically represents a pairing of an inhalation device with which the respiratory treatment, i.e., the inhalation process is performed and an external electronic device that are mutually configured to communicate with each other, such that inhalation process feedback can be provided also on said electronic device. The technical means to allow such mutual communication are the ones that are known to the skilled person and embrace wired and wireless communication means, like data-transfer cables, Bluetooth, any NFC or Wi-Fi hardware or the like. As part of the present disclosure and invention and preferably in the context of the present inhalation device system, it is also conceivable that inhalation process feedback is provided by means of e-mail or push-notification handled by the external electronic device.

The present disclosure and invention is not limited to the above defined methods and procedures of operating an inhalation device. The present disclosure and optionally the present invention also include an inhalation process in relation thereto. Said inhalation process comprises the following steps: performing an inhalation process to prevent or treat a respiratory disease or malfunction or a systemic disease using an inhalation device, monitoring the inhalation process with respect to pertinent inhalation process parameters and features and collecting respective data, concluding the inhalation process, evaluating the inhalation process retrospectively based on the monitoring results and the data obtained and collected during the inhalation process and outputting inhalation process feedback data that informs the user/patient and or a physician or caregiver about the retrospective quality of the inhalation process via suitable signals constituting the inhalation process feedback. Optionally, the inhalation process comprises the output of information of how to improve the inhalation process if it was found to be non-optimal. Further optionally, the user/patient is referred to a breath guiding procedure that is preferably facilitated and assisted by an electronic device, for example, a smartphone with a respectively configured computer program.

A yet further aspect of the present disclosure and invention is a computer program that is configured to facilitate the herein described methods and procedures. Specifically embraced is a computer program or app, respectively that is to be executed on respective means, i.e. computer processors of the inhalation device itself, but preferably of the external electronic device and configured to output the inhalation process feedback in any suitable form and in different degrees of detail. More specifically, the computer program is configured to output the inhalation process feedback on an electronic display device in graphical and/or audible form, for instance via generating a suitable graphical user interface, also in line with the examples given above and herein below. The output in the external computer program or app could either only be feedback regarding the most important inhalation process parameter or only regarding the least optimal inhalation process parameter or a list of all non-optimal inhalation process parameters or a list of all inhalation process parameters giving feedback on all of them whether optimal or not optimal or a list with prioritized feedback. Further embraced is a computer-readable storage medium on which the above computer program is stored and yet further a carrier containing the computer program, wherein the carrier is one of an electrical signal, optical signal, radio signal or computer-readable storage medium.

### EXAMPLES

In a preferred example of the present disclosure and invention, the inhalation device is a compact handheld device in which the signalling unit is integrated in the form of multi-colour light sources, preferably one or more LEDs. A user/patient performs an inhalation process using the inhalation device and once the inhalation process is completed, the signalling unit integrated into the inhalation device outputs respective signals to provide inhalation process feedback. As the first signal a green-illuminated small lung symbol is displayed on the shaft of the inhalation device that is suitable configured for signal output. Said symbol is then indicative of an optimal inhalation process, i.e., more specifically one or more pertinent inhalation process parameters were within a respectively pre-set range for a pre-set portion of the whole inhalation process. As the second signal, a yellow or red-illuminated small symbol, which could be a lung, a rewarding star or any other symbol associated with the quality of a breathing manoeuvre, is output instead and being indicative of a non-optimal inhalation process in retrospective. Surely, all other suitable and perceivable forms of signal output on the inhalation device are possible and this is only one non-limiting example.

A yet further non-limiting example is the provision of inhalation process feedback by means of an external electronic device preferably a smartphone or tablet computer. In such a scenario, a user/patient performs an inhalation process using the inhalation device, the inhalation process is monitored by the sensing unit and respective data collected until the end of the inhalation process. At the end of the process, the control unit generates and outputs the respective control signal for the signalling unit to output the first or the second signal. This procedure is basically identical to the one used in the above-mentioned example and in all the cases where the signalling unit is incorporated into inhalation device itself. However, in contrast, in the present example, the signalling unit is part of or represented by the external smartphone or tablet computer and electronic display devices, but also optionally all other signal output means therein are used to provide the inhalation process feedback. It is conceivable that the electronic display device also outputs the above described coloured lung symbols, but due to the larger technical capabilities of an electronic display device, many other and more elaborate ways of signal output are conceivable, for example, animated lung symbols could be displayed or written information provided or a voice output given or the inhalation process parameters to be optimized listed or displayed graphically, e.g., the not used up medication is shown as a non-empty ampoule symbol or the nose-breathing shown with a figure/symbol breathing through the nose. In the respective example, it is normally envisaged that the external electronic device, preferably the smartphone or tablet computer comprise a computer program preferably a bespoke app installed and executed thereon that realizes the software-side of the signal output and the provision of the inhalation process feedback. It could also be that the inhalation feedback is given to the patient after the inhalation as a push-notification if the app is not used by the patient at the moment to make him aware of the detailed feedback.

## Claims

1. A method of operating an inhalation device that includes:
a sensing unit configured to monitor an inhalation process, to measure inhalation process parameters and to collect data of and during the inhalation process, and
a control unit configured to receive and process the data collected by the sensing unit and to generate a control signal for a signalling device, wherein the method comprises:
monitoring an inhalation process comprising measuring inhalation process parameters and collecting data of and during the inhalation process,
generating and sending a control signal based on the collected data to a signalling unit configured to output a signal, preferably an optical, acoustic and/or a tactile signal, wherein
in response to said control signal, the signalling unit outputs a first signal after the inhalation process has been concluded if retrospectively one or more inhalation process parameters was/were within a pre-set range and outputs a second signal after the inhalation process has been concluded if retrospectively one or more inhalation process parameters was/were outside a pre-set range.

2. The method according to claim 1, wherein
the one or more inhalation process parameter is any one of the following group: respiratory minute volume, breathing frequency, peak inspiratory flow, tidal volume, regularity of tidal volume, regularity of breathing cycles duration, total duration of aerosol production, ratio of exhalations not done through the inhalation device, ratio of exhalation duration to inhalation duration, duration of a breathing pause after inhalation and after exhalation and degree of nebulization of the medicament or agent.

3. The method according to claim 1 or 2, wherein
the signalling unit comprises or consists of any one of the following: means to generate an optical signal; preferably a light signal, means to produce an acoustic signal; preferably a sound and/or a voice signal, means to produce a tactile signal; preferably a vibration signal and an electronic display device.

4. The method according any one of the preceding claims, wherein
the signalling unit is implemented in the inhalation device itself.

5. The method according to claim 4, wherein
the first signal is output as a green light signal and the second signal is output as a yellow or a red light signal, optionally wherein
the first light signal and the second light signal appear on the housing of the inhalation device.

6. The method according any one of claims 1 to 3, wherein
the signalling unit is implemented in a device different from and external to the inhalation device, preferably
in an electronic device further preferably with a computer program stored on the electronic device configured to generate a graphical user interface for outputting data of the inhalation process.

7. The method according to claim 6, wherein
the signalling unit is an electronic display device and the method further comprises:
in response to said control signal and after the inhalation process, the electronic display device displays summary and feedback information regarding the concluded inhalation process, wherein
the displayed information is to inform a user of the inhalation device about the quality of the concluded inhalation process and/or ways to improve the next inhalation process and/or to recommend an inhalation process guidance function for the next inhalation process.

8. An inhalation device optionally the one to carry out the method according to any one of claims 1 to 7, comprising:
a sensing unit configured to monitor an inhalation process and to measure and collect data of and during the inhalation process,
a signalling unit configured to output a signal, preferably an optical, acoustic and/or a tactile signal,
a control unit configured to receive and process the data collected by the sensing unit and to control the signalling unit based on said data, wherein
the control unit is further configured to control the signalling unit such that the signalling unit outputs a first signal after the inhalation process has been concluded if retrospectively one or more inhalation process parameters was/were within a pre-set range and outputs a second signal after the inhalation process has been concluded if retrospectively one or more inhalation process parameters was/were outside a pre-set range.

9. The inhalation device according to claim 8, wherein
the first signal is a green light signal and the second signal is a yellow or red light signal, optionally wherein
the first light signal and the second light signal appear on the housing of the inhalation device.

10. The inhalation device according to claim 8 or 9, wherein
the one or more inhalation process parameter is any one of the following group: respiratory minute volume, breathing frequency, peak inspiratory flow, tidal volume, regularity of tidal volume, regularity of breathing cycles duration, total duration of aerosol production, ratio of exhalations not done through the inhalation device, ratio of exhalation duration to inhalation duration, duration of a breathing pause after inhalation and after exhalation and degree of nebulization of the medicament or agent.

11. The inhalation device according to any one of the preceding claims, wherein
the signalling unit comprises any one of the following accommodated within the inhalation device: means to generate an optical signal; preferably a light signal, means to produce an acoustic signal; preferably a sound signal and/or a voice signal and means to produce a tactile signal; preferably a vibration signal and an electronic display device.

12. An inhalation device system, comprising:
an inhalation device, preferably the inhalation devices as used in any one of claims 1 to 7 or the inhalation device according to any one of claims 8 to 11 and
an electronic device that is different from and separate to the inhalation device, preferably a mobile phone, smartphone, smart watch, smart personal device or tablet computer, wherein
both the inhalation device and the electronic device comprise communication means configured to establish a wired or wireless communication connection between the inhalation device and the electronic device to exchange data on an inhalation process performed and concluded with the inhalation device.
